(19) Europäisches Patentamt
European Patent Office
Office européen des brevets

(11) EP 2 381 922 B1

(12) FASCICULE DE BREVET EUROPEEN

(45) Date de publication et mention
de la délivrance du brevet:
**24.10.2012 Bulletin 2012/43**

(51) Int Cl.:
***A61K 8/97*** *(2006.01)*      ***A61Q 5/02*** *(2006.01)*
***A61Q 5/00*** *(2006.01)*

(21) Numéro de dépôt: **09805745.8**

(22) Date de dépôt: **18.12.2009**

(86) Numéro de dépôt international:
**PCT/FR2009/052610**

(87) Numéro de publication internationale:
**WO 2010/076518 (08.07.2010 Gazette 2010/27)**

(54) **Utilisation d'une composition cosmétique capillaire comprenant un extrait d'acanthe**

Verwendung einer kosmetischen Haarpflegezusammensetzung enthaltend einen Akanthus Extakt

Use of a cometic hair care composition comprising an extract of acanthus

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **29.12.2008 FR 0807481**

(43) Date de publication de la demande:
**02.11.2011 Bulletin 2011/44**

(73) Titulaire: **Pierre Fabre Dermo-Cosmétique**
**92100 Boulogne-Billancourt (FR)**

(72) Inventeur: **FIORINI-PUYBARET, Christel**
**F-31500 Toulouse (FR)**

(74) Mandataire: **Rousseau, Pierrick Edouard**
**Pierre Fabre SA**
**Direction Propriété Intellectuelle**
**17, Avenue Jean Moulin**
**F-81106 Castres Cedex (FR)**

(56) Documents cités:
- **Dr. E.R.Pfaff: "Das menschliche Har" 1869, Verlag Otto Wigand , Leipzig , XP002540839page 101, le document en entier**
- **WERNER HUBER ET AL: "Annual Report 2006/2007 for the Project: Biological Corridor in the "Rainforest of the Austrians" Piedras Blancas National Park, Costa Rica" Internet citation, [Online] 26 juin 2007 (2007-06-26), pages 1-32, XP002571708 Extrait de l'Internet: URL:http://www.lagamba.at/researchdb/uploa d/files/annual%20report%202006-07_fertig_e nglisch.pdf> [extrait le 2010-03-08]**
- **M. O. NWOSU: "Plant resources used by traditional women as herbal medicines and cosmetics in Southeast-Nigeria" ÄRZTEZEITSCHRIFT FÜR NATURHEILVERFAHREN, vol. 41, no. 11, 2000, pages 760-764-767, XP002571709**
- **LOUKIS A ET AL: "Phytochemical investigation of acanthus mollis L" FITOTERAPIA, vol. 51, no. 4, 1 janvier 1980 (1980-01-01), pages 183-186, XP008109563 IDB HOLDING, MILAN, IT ISSN: 0367-326X cité dans la demande**
- **HOKPUTSA S ET AL: "Bioactive polysaccharides from the stems of the Thai medicinal plant Acanthus ebracteatus: their chemical and physical features", CARBOHYDRATE RESEARCH, PERGAMON, GB, vol. 339, no. 4, 15 March 2004 (2004-03-15), pages 753-762, XP004491927, ISSN: 0008-6215, DOI: 10.1016/J.CARRES.2003.11.022**

EP 2 381 922 B1

**Description**

L'invention se rapporte à l'utilisation d'une composition cosmétique capillaire comprenant un extrait d'acanthe pour améliorer la création des boucles et/ou à maintenir l'effet bouclant des cheveux.

L'Acanthe ou *Acanthus sp.* est une grande et belle plante vivace appartenant à la famille des Acanthacées. On la trouve principalement en Europe méditerranéenne et en Afrique septentrionale. Elle est cultivée comme plante ornementale la plupart du temps. Très décoratives, ses feuilles furent très utilisées par les Grecs comme modèle de frises décoratives ornant les colonnes du chapiteau corinthien. Les feuilles fraîches et les racines sont utilisées depuis l'Antiquité pour leurs propriétés médicinales. Depuis l'Antiquité, sa sève a la réputation d'être tonique et stimulante. Pline et Dioscoride la prescrivaient pour soigner les plaies (propriétés émollientes) et les dysenteries, diarrhées et fièvre (contient des mucilages) (LOUKIS et PHILIANOS, 1980 - Phytochemical investigation of Acanthus molis L. Fitoterapia, 51 (4), 183-186). Par voie orale, elle était recommandée comme diurétique et antidysentérique. En topique, elle était réputée adoucissante et émolliente (brûlures, fractures, affections buccales). De nos jours, son usage est relativement limité, elle est encore employée en homéopathie pour soigner la toux.

L'acanthe est une plante herbacée vivace, mesurant 30 à 80 cm de haut, ayant un rhizome traçant de couleur blanchâtre, très long, pouvant atteindre 3 cm de diamètre et muni de nombreuses radicelles. Le port est une touffe évasée pouvant atteindre 90 cm de large. Les tiges sont circulaires et solides. Les feuilles sont très grandes, un peu molles, d'un beau vert foncé brillant, découpé, caduques ; les feuilles sont simples, opposées, profondément lobées, dentées et portées par des tiges charnues. Le pétiole est long.

La feuille libère lorsqu'on presse son pétiole un « suc » translucide et visqueux à qui l'on attribue les bienfaits de la plante. Ce mucilage, également présent dans les autres parties de la plante comme les racines, les fleurs , les tiges, est constitué, comme la plupart des gommes et des exsudats végétaux, de composés issus de la condensation de glucides (polysaccharides). On trouve également dans la feuille des flavonoïdes (flavones méthoxylées : hispiduline), des acides phénoliques (acides caféique, acide chlorogénique), des glycoprotéines et acides aminés.

Les fleurs sont blanches et regroupées sur une grande hampe florale érigée (1 à 2 m). Les hampes florales portent des épis de fleurs tubulaires de 5 à 6 cm de long, groupées par 2 ou par 4.

Les fruits sont de longues capsules brillantes, ovoïdes, formées de 2 valves, libérant 2 à 4 grosses graines.

Dr. E.R.Pfaff: "Das menschliche Har", 1869, Verlag Otto Wigand, Leipzig, page 101, divulgue l'utilisation d'un extrait d'acanthe en capillaire.

Annual Report 2006/2007 for the Project Biological Corridor in the "Rainforest of the Austrians" Piedras Blancas National Park, Costa Rica, décrit un shampoing à base d'un extrait d'acanthe.

M. O. Nwosu: "Plant resources used by traditional women as herbal medicines and cosmetics in Southeast-Nigeria", Ärztezeitschrift für Naturheilverfahren, vol. 41, no. 11, 2000 , pages 760,764,767, décrit l'utilisation cosmétique pour la peau d'un extrait d'acanthe.

La demanderesse a mis en évidence une nouvelle valorisation de l'*Acanthus mollis* en cosmétique capillaire où de façon inattendue et surprenante, l'*Acanthus mollis* a montré de bonnes capacités à créer des boucles plus serrées et à maintenir l'effet bouclant des cheveux.

On peut raisonnablement envisager que les bénéfices de la présente invention aujourd'hui démontrés par les inventeurs à partir d'*Acanthus mollis,* compte tenu de la facilité d'approvisionnement en végétal *Acanthus mollis* dans notre pays, puissent s'étendre au genre Acanthe ou Acanthus sp.

Parmi les espèces d'Acanthe les plus réputées pour leur richesse en mucilage et/ou facilement disponibles et abondantes pour une mise en culture à visée industrielle, on peut citer préférentiellement: *Acanthus mollis, Acanthus ilicifolius, Acanthus ebractaetus, Acanthus montanus, Acanthus arboreus, Acanthus dioscoridis, Acanthus hungaricus, Acanthus senii, Acanthus spinosus, Acanthus syriacus* et *Acanthus carduaceus.*

Plus préférentiellement, les espèces d'Acanthe seront choisies parmi le groupe constitué de *Acanthus mollis, Acanthus ilicifolius, Acanthus ebractaetus,* et *Acanthus montanus.*

De façon encore plus préférentielle, l'acanthe utilisée dans le cadre de la présente invention est l'espèce *Acanthus mollis.*

L'objet de la présente invention est l'utilisation d'une composition cosmétique capillaire comprenant un extrait d'acanthe présentant une fraction massique en mucilages, exprimée en sucres réducteurs totaux, comprise entre 1 % et 80 % ; et de façon avantageuse supérieure à 30 % pour améliorer la création des boucles et/ou maintenir l'effet bouclant de cheveux.

Par « extrait d'Acanthe », on entend au sens de la présente invention :

- la solution obtenue après extraction du végétal *Acanthus sp.* par un solvant
- ou le jus obtenu par pressage ou par extrusion.

Les feuilles, les tiges, les fleurs ou les racines ou un mélange de ces parties fraîches de préférence peuvent être soit extraites par un solvant ou soit pressées pour en exprimer le jus.

Dans un mode de réalisation particulier de l'invention, les parties de plante récoltées sont congelées avant d'être stockées.

Ledit extrait d'acanthe selon la présente invention peut être obtenu comme suit :

- Dans le premier cas de figure, les feuilles, les tiges, les fleurs ou les racines ou un mélange de ces parties sont de préférence préalablement broyées puis extraites avec un solvant qui peut être de l'eau ou un mélange d'eau et de solvants organiques miscibles à l'eau tels qu'un alcool (méthanol, éthanol ...), une cétone (acétone) ... L'extraction est réalisée dans un ratio plante/solvant compris entre environ 1/1 et environ 1 /20 et peut être renouvelée 2 à 3 fois. La température du solvant d'extraction peut être égale à la température ambiante ou supérieure, pouvant atteindre la température d'ébullition du solvant engagé. Le temps de contact de la plante avec le solvant est compris entre environ 30 min et environ 72 heures.
- Dans le second cas de figure le jus des feuilles, tiges, fleurs ou racines ou un mélange de ces parties fraîches de préférence peut être obtenu par pressage ou par extrusion.

On procède ensuite à une séparation solide/liquide, la plante étant séparée du solvant par filtration ou centrifugation.

Le filtrat obtenu est concentré sous vide et à une température comprise entre la température ambiante et la température d'ébullition. Le concentrat subit une étape de stérilisation par traitement thermique et de stabilisation par addition d'alcool puis séché par évaporation totale du solvant d'extraction, ou conservé en l'état sous forme d'extrait fluide. Le séchage de l'extrait final peut être réalisé par lyophilisation ou par des moyens de séchage plus classiques connus de l'homme du métier (nébulisation, étuve, sécheur à palette ...).

L'extrait peut être stabilisé par addition d'un antioxydant ou d'un conservateur comme par exemple l'acide ascorbique, l'acide citrique à des quantités comprises entre environ 0,05 et environ 1 g pour 100 g d'extrait sec.

L'extrait d'acanthe se caractérise chimiquement par sa teneur en mucilages, en acides aminés et en protéines (Van Holst et Clarke, 1985 - Quantification of Arabinogalactan-Protein in Plant Extracts by single Radial Gel Diffusion - Analytical Biochemistry 148, 446-450).

A l'état naturel, la plante contient une fraction massique en mucilages comprise entre environ 5 % et environ 10 % environ.

Dans le cadre de la présente invention, on utilisera préférentiellement un extrait d'acanthe plus ou moins purifié.

La fraction massique en mucilages dudit extrait, exprimée en sucres réducteurs totaux, est comprise entre environ 1% et environ 80 % ; de façon plus avantageuse comprise entre environ 15% et environ 80% et de façon encore plus avantageuse supérieure à 30 % (soit comprise entre environ 30% et environ 80%). Dans le cas d'un jus d'acanthe, la teneur en sucres réducteurs est généralement de l'ordre de 5 % environ.

Le dosage des sucres réducteurs peut se faire de toute manière classique bien connue de l'homme du métier.

La méthode de dosage des sucres réducteurs classiquement utilisée consiste en un dosage colorimétrique des sucres réducteurs par l'acide 3,5-dinitrosalicylique (DNS) par rapport au galactose. Les résultats sont exprimés en pourcentage de sucres réducteurs par rapport au galactose. La méthode de dosage est rappelée ci-après :

**Dosage des sucres réducteurs totaux exprimés en galactose en p. cent (m/m)**

Préparations des réactifs :

*- Hydroxyde de sodium à 2N*

*- Réactif au DNS :*

*Solution de ditartrate de sodium et potassium (solution 1) :*
Dissoudre 15 g de ditartrate de sodium et de potassium dans 25.0 ml d'eau. Agiter jusqu'à dissolution complète.
*Solution de Dinitrosalicylique (DNS) (solution 2)* :
Peser 0,5 g de DNS dans une fiole de 50 ml.
Mettre en solution dans 10 ml d'hydroxyde de sodium à 2N en chauffant légèrement sous agitation magnétique à 30-35° C pendant 1 heure.

Puis Ajouter la solution 1 à la solution 2

Chauffer légèrement (30-35° C) sous agitation magnétique jusqu'à dissolution complète *(temps de dissolution environ 6h)*

Préparation de la gamme étalon :

Témoin 1 : Dissoudre 7.5 mg de galactose dans 20 ml d'eau
Témoin 2 : Dissoudre 10.0 mg de galactose dans 20 ml d'eau
Témoin 3 : Dissoudre 12.5 mg de galactose dans 20 ml d'eau

Préparation de solutions essais :

Dissoudre 25.0 mg d'extrait, exactement pesé, dans 20.0 ml d'eau. Réaliser 2 essais.

Dosage

Dans une série de tubes gradués de 10 ml introduire :

|  | T1 | T2 | T3 | Essai | Blanc |
|---|---|---|---|---|---|
| Solutions témoins (ml) | 1.0 | 1.0 | 1.0 | / | / |
| Solutions essai (ml) | / | / | / | 1.0 | / |
| Eau ultra pure (ml) | 1.0 | 1.0 | 1.0 | 1.0 | 2.0 |
| Réactif au DNS (ml) | 1.0 | 1.0 | 1.0 | 1.0 | 1.0 |

Agiter puis placer 5 minutes au bain-marie à 95 ° C.
Refroidir environ 10 min dans un bain de glace et compléter jusqu'à 10 ml avec de l'eau.
Mesurer l'absorbance à 500 nm des différentes solutions contre le blanc.

Calcul

Tracer la courbe d'étalonnage et en déduire la concentration en sucres réducteurs totaux ($Q_{SRT}$), exprimée en galactose dans les solutions essais.
Le titre en sucres réducteurs totaux ($T_{SRT}$) en p. cent (m/m) de l'extrait est donné par la formule suivante :

$$T_{SRT}\ (\%) = \frac{\text{Qsrt x 100 x 20}}{\text{Pe2}}$$

avec $Q_{SRT}$ en mg/ml
Pe2 en mg (prise d'essai en mg)
Un autre objet de la présente invention concerne également une composition cosmétique à base d'acanthe en tant que principe actif et au moins un excipient cosmétiquement acceptable.
Préférentiellement, l'acanthe se présente sous la forme d'un extrait présentant une fraction massique en mucilages, exprimée en sucres réducteurs totaux, comprise entre 1 % et 80 %.
De façon avantageuse, la composition cosmétique selon la présente invention comprend une quantité d'extrait sec d'acanthe, à titre de principe actif, comprise entre 0,010 g et 2 g; et plus préférentiellement entre 0,10 g et 0,70 g pour 100 g de la dite composition.
La composition cosmétique selon la présente invention peut avantageusement se présenter sous toutes les formes galéniques normalement utilisées dans le domaine cosmétique pour une application topique. Préférentiellement, la forme topique peut être notamment sous forme de: shampoing, baume, gel, lotion, mousse, spray, crème.
On distingue ainsi des produits formulés pour être rincés et d'autres non.
Dans un mode de réalisation particulier de la présente invention, la dite quantité d'extrait sec d'acanthe est comprise entre 0,3 et 1 g et préférentiellement égale à environ 0,5 g pour 100 g de composition cosmétique destinée à être rincée après son application.
Selon un autre mode de réalisation particulier de l'invention, ladite quantité d'extrait sec d'acanthe est comprise entre environ 0,1 et 0,3 g pour 100 g de composition cosmétique qui ne sera pas rincée après son application sur les cheveux.

L'extrait d'acanthe peut également être associé à d'autres actifs tels que l'huile d'avocat aux propriétés nutritives.

La composition cosmétique selon l'invention comprend, en outre, des excipients usuels cosmétiquement compatibles.

Les excipients usuels compatibles à la composition cosmétique capillaire peuvent être tout excipient parmi ceux connus de l'homme du métier en vue d'obtenir une composition cosmétique pour une application topique sous les formes telles que décrites ci-dessus.

La composition cosmétique selon l'invention peut en particulier contenir des additifs et aides à la formulation, tels que des tensio-actifs de type émulsionnant, nettoyant, moussant, etc..., des agents complexants, des épaississants, des gélifiants, des stabilisants, des conservateurs dont des antimicrobiens et des antioxydants, des conditionneurs, des acidifiants, des alcalinisants, des émollients, des solvants, des colorants, des parfums.

La composition peut comprendre d'autres composés utiles au conditionnement du cheveu, tels que des agents de coloration, de brillance par exemple.

La présente invention concerne l'utilisation d'une composition cosmétique capillaire comprenant un extrait d'acanthe pour améliorer, faciliter la formation des boucles et/ou maintenir l'effet bouclant des cheveux.

L'Acanthe possède un effet bouclant et améliore le maintien des boucles. Les meilleurs résultats de l'effet bouclant ont été remarqués lorsque l'acanthe se présente sous la forme d'un extrait d'acanthe présentant une fraction massique en mucilages, exprimée en sucres réducteurs totaux, comprise entre 1 % et 80%.

La présente invention concerne l'utilisation d'une composition cosmétique capillaire comprenant un extrait d'acanthe pour améliorer la formation des boucles et/ou maintenir l'effet bouclant des cheveux ; caractérisée en ce que l'acanthe se présente sous la forme d'un extrait d'acanthe présentant une fraction massique en mucilages, exprimée en sucres réducteurs totaux, comprise entre 1 % et 80 % et encore plus préférentiellement supérieure à 30%.

Ledit extrait d'acanthe, uitlisé dans le cadre de la présente invention, contient en outre des acides aminés et des protéines.

Enfin, l'acanthe sera avantageusement choisie parmi le groupe constitué des espèces *suivantes : Acanthus mollis, Acanthus ilicifolius, Acanthus ebractaetus, Acanthus montanus, Acanthus arboreus, Acanthus dioscoridis, Acanthus hungaricus, Acanthus senii, Acanthus spinosus, Acanthus syriacus* et *Acanthus carduaceus.*

Les compositions selon la présente invention redonnent forme et matière aux cheveux bouclés, ondulés ou frisés.

L'efficacité de l'extrait d'acanthe sur les boucles des cheveux se traduit par :

- la formation de boucles de cheveux plus serrées (mèches de cheveux significativement plus courtes, effet bouclant optimisé);
- une meilleure tenue des boucles de cheveux (boucles se détendant moins vite).

Les préparations et les compositions suivantes sont citées à titre d'exemples illustratifs et non limitatifs.

<u>EXEMPLES DE PREPARATION DE L'EXTRAIT VEGETAL</u>

**Exemple 1** : Préparation d'un extrait *d'Acanthe*

Les feuilles fraîches congelées *d'Acanthus mollis* L. sont broyées puis extraites sous agitation avec 7 volumes d'eau à 80°C pendant 48 heures.

La plante est séparée du solvant par filtration.

Le filtrat obtenu est concentré sous vide à une température de 80°C jusqu'à 30 % d'extrait sec. Le concentrât est stérilisé à 121 °C pendant 20 min et stabilisé par addition d'éthanol.

L'extrait final est standardisé à 70 % d'extrait sec par addition de maltodextrine. L'extrait obtenu contient environ 35 g de sucres réducteurs totaux exprimés en galactose pour 100g d'extrait sec.

**Exemple 2** : Préparation d'un extrait *d'Acanthe*

Les feuilles entières fraîches congelées d'Acanthus *mollis* sont broyées puis introduite dans une extrudeuse afin d'en recueillir le jus. Ce dernier est essoré afin d'éliminer les éventuels débris de plante stérilisé par chauffage à 121 °C pendant 20 min puis stabilisé par addition d'éthanol. Après évaporation de l'alcool, le stabilisât alcoolique est congelé puis mis à sec par lyophilisation. L'extrait obtenu contient environ 50 g de sucres réducteurs totaux exprimés en galactose pour 100 g d'extrait sec.

EXEMPLE DE COMPOSITIONS COSMETIQUES A BASE D'ACANTHE

**Exemple 1: BAUME A RINCER**

- EXTRAIT SEC ACANTHE 0,3 à 1 g
- HUILE AVOCAT 1,0 g
- ALCOOL CETEARYL / CETEARETH 33 8,0 g
- ALCOOL CETEARYLIQUE 2,0 g
- AMODIMETHICONE SOLUTION 8,0 g
- BEHENTRIMONIUM CHLORURE 3,0 g
- POLYQUATERNIUM 11 0,75 g
- BUTYLHYDROXYTOLUENE 0,02 g
- EDTA 2 Na 0,2 g
- VINAIGRE NATUREL 5,0 g
- Parfum(s) qs
- Colorant(s) qs
- Conservateur(s) qs
- EAU PURIFIEE qsp 100,0 g

**Exemple 2: SHAMPOING**

- EXTRAIT SEC ACANTHE 0,3 à 1 g
- HUILE AVOCAT 0,1 g
- GUAR hydroxypropyltrimonium chloride 0,5 g
- PROPYLENE GLYCOL 1,0 g
- LAURETH SULFATE SODIUM 9,5 g
- LAURYL BETAINE 1,7 g
- LAURYL POLYGLUCOSIDE 50% 5,0 g
- CETEARETH-60 MYRISTYL GLYCOL 1,0 g
- EDTA SODIUM 2 Na 0,2 g
- CETRIMONIUM CHLORURE 1,5 g
- Agent nacrant qs
- Conservateur(s) qs
- Parfum(s) qs
- Colorant(s) qs
- CITRIQUE ACIDE MONOHYDRATE qsp pH = 5,0
- EAU PURIFIEE qsp 100,0 g

**Exemple 3: SOIN SANS RINCAGE**

- EXTRAIT SEC ACANTHE 0,1 à 0,3 g
- HUILE AVOCAT 0,5 g
- POLYMERE ACRYLIQUE 0,2 g
- POLYSACCHARIDE NATUREL 0,1 g
- Alcool cetylstéarylique /alcool cetylstéarylique éthoxylé (30 moles) 3,0 g
- PALMITATE ETHYLHEXYLE 10,0 g
- CETEARYLIQUE ALCOOL 3,0 g
- AMODIMETHICONE SOLUTION 2,0 g
- POLYQUATERNIUM 68 2,5 g
- CETRIMONIUM CHLORURE 0,5 g
- EDTA SODIUM 2 Na 0,2 g
- Conservateur(s) qs

- Parfum(s) qs
- EAU PURIFIEE qsp 100,0 g

TEST BIOMETROLOGIQUE SUR MECHES DE CHEVEUX

Une étude d'efficacité en cosmétique capillaire a permis d'évaluer l'activité d'une solution aqueuse à 1 % d'un extrait d'acanthe obtenu par le procédé décrit dans l'exemple 1 sur le maintien des boucles de cheveux en comparaison avec un témoin non traité.

Pour cela, les mèches sont préalablement lavées de façon standardisée puis séchées également de façon standardisée (séchage pendant 18 heures sous conditions contrôlées de température et d'humidité : T° = 21 ± 1 °C, humidité relative = 50 ± 5%). Ces mèches sont ensuite immergées dans la solution aqueuse à base d'acanthe (groupe traité) ou dans de l'eau (groupe témoin) pendant 3 minutes.

Les mèches sont alors placées sur un dispositif permettant la création des boucles (bigoudis de 16 mm de diamètre) pendant 18 heures dans les conditions de température et de d'humidité suivantes : T° = 21 ± 1 °C, humidité relative = 50 ± 5% ; puis 90 minutes à T° = 25 ± 1°C, humidité relative = 75 ± 5%.

Les bigoudis sont ensuite retirés et la longueur des mèches est mesurée dans le groupe traité et dans le groupe témoin à différents temps après retrait des bigoudis :

- première mesure immédiatement après retrait des bigoudis (t = 0)
- puis à t = 30, 90 et 5 heures

Pour réaliser ces mesures, les mèches des cheveux sont suspendues sur un appareil qui en détermine automatiquement la longueur.

L'activité sur le maintien des boucles des cheveux est évaluée à l'aide du paramètre nommé « curl retention » et noté CR, exprimé en % tel que :

$$CR = \frac{l_f - l_{ti}}{l_f - l_0} \times 100$$

avec

CR = Curl Retention (%)
$l_f$ = longueur initiale de la mèche de cheveux non bouclée (cm)
$l_{ti}$ = longueur de la mèche de cheveux au temps t, (cm)
$l_{t0}$ = longueur de la mèche de cheveux au temps t = 0 (cm)

RESULTATS:

Tableau 1 : Longueurs des mèches de cheveux et Paramètre CR obtenus à T0, 30, 90 et minutes après retrait des bigoudis.

| | | t avant traitement | t0 | t 30 min | t 90 min | t 300 min |
|---|---|---|---|---|---|---|
| **Groupe traité** | **Longueur moyenne (mm)** | 216 mm | 99.91 ±5.92 | 117.16 ±5.52 | 127.99 ±4.82 | 134.58 ±5.52 |
| | **CR moyen (%)** | - | 100% | 85% | 76% | 70% |

(suite)

|  |  | t avant traitement | t0 | t 30 min | t 90 min | t 300 min |
|---|---|---|---|---|---|---|
| Groupe témoin | Longueur moyenne (mm) | 216 mm | 109.67 ±5.56 | 132.17 ±5.85 | 145.70 ±4.22 | 153.43 ±3.46 |
| | CR moyen (%) | - | 100% | 79% | 66% | 59% |

L'analyse des résultats à t 0 (immédiatement après retrait des bigoudis) montre des mèches significativement plus courtes dans le groupe traité par l'extrait d'acanthe en comparaison au groupe témoin ($l_{t0}$ = 109.67 ±5.56 mm).

Cette différence traduit l'effet bouclant de l'acanthe (p=0.02).

L'analyse des résultats montre également un maintien des boucles significativement plus important à t 30, t 90 et t 300 minutes dans le groupe traité par l'extrait d'acanthe en comparaison au groupe témoin (p=0.002 à t 30 et p<0.001 à t 90 et t 300).

CONCLUSION :

Dans les conditions de réalisation de cette étude, et après une seule application, l'effet de l'extrait d'acanthe sur la formation et la tenue des boucles a été démontré.

## Revendications

1. Utilisation d'une composition cosmétique capillaire comprenant un extrait d'acanthe à titre de principe actif, présentant une fraction massique en mucilages, exprimée en sucres réducteurs totaux, comprise entre 1 % et 80 % ; et au moins un excipient cosmétiquement acceptable pour améliorer la création des boucles et/ou maintenir l'effet bouclant des cheveux.

2. Utilisation selon la revendication 1 **caractérisée en ce que** l'acanthe se présente sous la forme d'un extrait d'acanthe présentant une fraction massique en mucilages, exprimée en sucres réducteurs totaux, supérieure à 30 %.

3. Utilisation selon la revendication 1 **caractérisée en ce que** l'extrait d'acanthe contient en outre des acides aminés et des protéines.

4. Utilisation selon la revendication 1 **caractérisée en ce que** la composition comprend une quantité d'extrait sec d'acanthe comprise entre 0,01 g et 2 g pour 100 g de ladite composition.

5. Utilisation selon la revendication 1 **caractérisée en ce que** la composition se présente sous la forme d'un shampoing, baume, gel, lotion, mousse, spray, crème.

6. Utilisation selon l'une quelconque des revendications 1 à 5 **caractérisée en ce que** l'acanthe est choisie parmi les espèces *Acanthus mollis, Acanthus ilicifolius, Acanthus ebractaetus, Acanthus montanus, Acanthus arboreus, Acanthus dioscoridis, Acanthus hungaricus, Acanthus senii, Acanthus spinosus, Acanthus syriacus* et *Acanthus carduaceus.*

## Claims

1. Use of a hair cosmetic composition comprising an acanthus extract as active principle, exhibiting a fraction by weight of mucilages, expressed as total reducing sugars, of between 1% and 80%, and at least one cosmetically acceptable excipient for improving the creation of curls and/or maintaining the curling effect on the hair.

2. Use according to Claim 1, **characterized in that** the acanthus is provided in the form of an acanthus extract exhibiting a fraction by weight of mucilages, expressed as total reducing sugars, of greater than 30%.

**3.** Use according to Claim 1, **characterized in that** the acanthus extract additionally comprises amino acids and proteins.

**4.** Use according to Claim 1, **characterized in that** the composition comprises an amount of acanthus on a dry basis of between 0.01 g and 2 g per 100 g of the said composition.

**5.** Use according to Claim 1, **characterized in that** the composition is provided in the form of a shampoo, balm, gel, lotion, foam, spray or cream.

**6.** Use according to any one of Claims 1 to 5, **characterized in that** the acanthus is chosen from the species *Acanthus mollis, Acanthus ilicifolius, Acanthus ebractaetus, Acanthus montanus, Acanthus arboreus, Acanthus dioscoridis, Acanthus hungaricus, Acanthus senii, Acanthus spinosus, Acanthus syriacus* and *Acanthus carduaceus.*

**Patentansprüche**

**1.** Verwendung einer Haarkosmetikzusammensetzung, die einen Akanthusextrakt als Wirkstoff mit einem Massenanteil an Schleimstoffen, ausgedrückt als reduzierende Gesamtzucker, von zwischen 1% und 80% sowie mindestens einen kosmetisch unbedenklichen Grundstoff umfasst, zur Verbesserung der Erzeugung von Locken und/oder Aufrechterhaltung des Lockeneffekts des Haars.

**2.** Verwendung nach Anspruch 1, **dadurch gekennzeichnet, dass** der Akanthus in Form eines Akanthusextrakts mit einem Massenanteil an Schleimstoffen, ausgedrückt als reduzierende Gesamtzucker, von oberhalb 30% vorliegt.

**3.** Verwendung nach Anspruch 1, **dadurch gekennzeichnet, dass** der Akanthusextrakt weiterhin Aminosäuren und Proteine enthält.

**4.** Verwendung nach Anspruch 1, **dadurch gekennzeichnet, dass** die Zusammensetzung eine Menge an Akanthustrockenextrakt von zwischen 0,01 g und 2 g pro 100 g Zusammensetzung umfasst.

**5.** Verwendung nach Anspruch 1, **dadurch gekennzeichnet, dass** die Zusammensetzung in Form eines Shampoos, eines Balsams, eines Gels, einer Lotion, eines Schaums, eines Sprays oder einer Creme vorliegt.

**6.** Verwendung nach einem der Ansprüche 1 bis 5, **dadurch gekennzeichnet, dass** der Akanthusextrakt aus den Arten *Acanthus mollis, Acanthus ilicifolius, Acanthus ebractaetus, Acanthus montanus, Acanthus arboreus, Acanthus dioscoridis, Acanthus hungaricus, Acanthus senii, Acanthus spinosus, Acanthus syriacus* und *Acanthus carduaceus* ausgewählt ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **LOUKIS ; PHILIANOS.** Phytochemical investigation of Acanthus molis L. *Fitoterapia,* 1980, vol. 51 (4), 183-186
- **DR. E.R.PFAFF.** Das menschliche Har. Verlag Otto Wigand, 1900, 101
- **M. O. NWOSU.** Plant resources used by traditional women as herbal medicines and cosmetics in Southeast-Nigeria. *Ärztezeitschrift für Naturheilverfahren,* 2000, vol. 41 (11), 760, 764, 767
- **VAN HOLST ; CLARKE.** Quantification of Arabinogalactan-Protein. *Plant Extracts by single Radial Gel Diffusion - Analytical Biochemistry,* 1985, vol. 148, 446-450